# EUROPEAN PATENT SPECIFICATION

(11) **EP 3 939 644 B1**
(45) Date of publication and mention of the grant of the patent: **16.07.2025**
(21) Application number: 19918641.2
(22) Date of filing: 13.03.2019
(51) Int. Cl.: A61M 16/20, A61M 16/00

(54) **TRANSPORTABLE LUNG VENTILATOR**
TRANSPORTIERBARES LUNGENBEATMUNGSGERÄT
VENTILATEUR PULMONAIRE DE TRANSPORT

(43) Date of publication of application: 19.01.2022
(73) Proprietor: Magnamed Tecnologia Médica S/A, 04004-030 São Paulo - SP (BR)
(72) Inventor: SUZUKI, Tatsuo, 04026-030 São Paulo (BR); MIYAGI KINJO, Toru, 04282-000 São Paulo (BR); UEDA, Wataru, 04006-052 São Paulo (BR)
(74) Representative: Plasseraud IP
(86) International application number: PCT/BR2019/050082
(87) International publication number: WO 2020/181343

(56) References cited:
- EP-A1- 0 347 015
- AU-B2- 2006 344 054
- BR-A2- PI1 002 124
- BR-A2- PI1 002 124
- US-A1- 2018 036 199
- US-A1- 2018 036 199

## Description

### FIELD OF THE INVENTION

The present invention relates to a transport pulmonary ventilator and, more specifically, to a pulmonary ventilator that allows automatic selection between oxygen and air as the motive gas of a venturi type ventilation system.

### BACKGROUND OF THE INVENTION

Different types of pulmonary ventilators for ventilation of patients with respiratory failure are known in the state of the art. Transport ventilators are non-stationary ventilators, which can be used for inter or intra-hospital transport of the patient. Thus, this type of ventilator tends to be smaller and have a more resistant body, being able to be used in transport means such ambulances or aircrafts, or even transported next to stretchers or attending devices in rescues.

The development of transport ventilators presents a technical challenge. On the one hand, the ventilators should be robust enough to handle vibrations from transport, on the other hand, should include the greatest amount of resources possible to fully meet the needs of the patient.

The differences between patients' respiratory needs are even more evident when considering the needs of adult patients and neonatal patients.

Among the variables associated with ventilation support is the fraction of inspired oxygen, also known as FiO₂, which represents the concentration of oxygen that is offered to the patient.

Conventional transport ventilators use venturi system to drag ambient air, with compressed oxygen being used as the motive gas. Thus, in this type of ventilator, the venturi system causes the oxygen under pressured to generate a vacuum that allows the dragging of ambient air in order to generate the gas mixture used in pulmonary ventilation.

These known solutions, however, have a deficiency of yield: when the oxygen flow is low, the dragging of ambient air is low; meaning, the air is not sufficiently sucked in. This low efficiency of air suction for low flow rates causes the FiO₂ to rise in the mixture.

Excess oxygen can cause damage, especially in neonates and mainly in preterms. The high concentration of oxygen in the blood, for example, can lead to abnormal retinal vessel growth (retinopathy of prematurity) and the prolonged exposure to high oxygen concentrations can lead to bronchopulmonary dysplasia, pulmonary atelectasis, and other problems.

Thus, for these patients, ventilation with air without the addition of oxygen (21% O₂) is desired. It occurs that even in ventilators that use optimized venturi systems to obtain the maximum yield in the low flow range used in neonates and preterms, the construction of the system keeps from reaching a percentage of 21% of oxygen.

A known solution of the state of the art for the high FiO₂ problem is the use of a pneumatic air/O₂ blender. This type of blender, which is coupled to the oxygen inlet, allows gas adjustment from 21% to 100%.

However, this known solution also has drawbacks, since the cost of the air/O₂ blender is very high, the device volume is significant, and the use of the pneumatic blender requires the use of two gas cylinders, the compressed air cylinder and the compressed oxygen cylinder, causing transport difficulties. In addition to the high cost and difficulty generated by the cylinders, the pneumatic blender requires the manual adjustment of FiO₂, which is uncomfortable and causes operational risks.

Additionally, even if the compressed air cylinder could be replaced by an air compressor associated with the pneumatic blender, the weight of a compressor is too large to be transported easily. Besides that, the use of the compressor in the ventilator requires the use of a reservoir to supply the demand peaks of the air flow and a dehumidifier due to the generation of water by natural humidity present in the ambient air that condenses when compressed.

Another known solution involves the use of internal turbines. In this solution, the ventilator uses an internal turbine that generates airflow with enough pressure to ventilate the lung. Although the use of a turbine with the addition of pure oxygen through a proportional valve in the gas that goes to the patient allows the adjustment of the oxygen concentration from 21% to 100%, it is a high cost solution, which requires a greater consumption and results in a ventilator with greater weight and size. In addition, the turbine generates noise and engine heat, which, if not treated properly, can lead to overheating of the air going to the patient.

Thus, there remains in the state of the art the need for a transport ventilator that is capable of meeting the needs of both adult and neonatal patients, allowing adjustment of oxygen concentration so that the fraction of inspired oxygen is suitably reached in each situation.

BR PI1002124 A2 discloses a transport ventilator usable for neonatal to adult patients. US 2018/036199 A1 discloses a method and apparatus for providing percussive ventilation therapy to a patient airway.

### OBJECTIVES OF THE INVENTION

It is an objective of the present invention to provide a compact and efficient transport ventilator, which is capable of meeting the needs of both adult and neonatal patients.

Another objective of the present invention is to provide a transport ventilator capable of adjusting oxygen concentration so that the fraction of inspired oxygen is suitably reached in each situation.

It is another objective of the present invention to provide a transport ventilator which allows an automatic selection between oxygen and air as the motive gas for a venturi type ventilation system.

Another objective of the present invention is to provide a transport ventilator adapted to utilize a small motive air source coupled to the ventilator body.

It is yet another objective of the present invention to provide a transport ventilator which dispenses the use of internal reservoir of air and dryer even when air compressor is used.

### BRIEF DESCRIPTION OF THE INVENTION

The invention is defined by the appended claims. The present invention achieves these and other objectives through a transport ventilator characterized by comprising:
a monobloc body with at least one medical oxygen inlet; at least one compressed air inlet, and at least one ambient air inlet;
at least one selector valve integrated to the monobloc body and adapted to selectively allow the passage of medical oxygen or compressed air;
a venturi system, internal to the monobloc body, which comprises an injector nozzle and a venturi tube, the injector nozzle receiving the medical oxygen or compressed air that passes through the selector valve and generating a jet towards the venturi tube, the jet dragging ambient air through the inlet of ambient air to generate a mixture of gases to be ventilated to a patient;
a sensor of fraction of inspired oxygen that measures the concentration of oxygen in the gas mixture; and
an electronic control system that controls the selector valve to allow the passage of medical oxygen or compressed air based on the oxygen concentration measured by the sensor of fraction of inspired oxygen.

With this construction, the transport ventilator of the present invention allows the oxygen concentration in the inspired gas to be adjusted to values between 21% and 100% by the electronic control of the selector valve.

Preferably, the inlet of compressed air is supplied by a small compressed air source coupled to the monobloc body. The small compressed air source may be, for example, a mini compressor coupled to the monobloc body or a small air cylinder coupled to the monobloc body.

In the preferred embodiment of the present invention, the medical oxygen inlet is an oxygen pressure regulating valve, the compressed air inlet is an air pressure regulating valve, and the ambient air inlet is a one-way valve associated with a filter.

The transport ventilator further comprises a proportional valve which receives the oxygen or compressed air that passes through the selector valve and directs it to the injector nozzle; a proportional oxygen valve that allows the passage of medical oxygen from the oxygen pressure regulating valve directly to the gas mixture to be ventilated; and a proportional air valve that allows the passage of compressed air from the air pressure regulating valve directly to the gas mixture to be ventilated.

Thus, the electronic control system can open and close the proportional oxygen valve and proportional air valve based on the measurement of the output flow of gas mixture measured by a flow sensor, so that it is possible to complete the current volume required for the adjusted FiO₂ in the ventilator.

### BRIEF DESCRIPTION OF DRAWINGS

The present invention will be described in more detail below along with references to the attached drawings, in which:
**Figure 1** **-** is a perspective view of the transport ventilator according to the preferred embodiment of the present invention;
**Figure 2** **-** is a second perspective view of the transport ventilator according to the preferred embodiment of the present invention;
**Figure 3** **-** is a cross-sectional view of the transport ventilator according to the preferred embodiment of the present invention; and
**Figure 4** **-** is a schematic illustration of the integrated electronic blender comprised by the transport ventilator according to the preferred embodiment of the present invention.

### DETAILED DESCRIPTION OF THE INVENTION

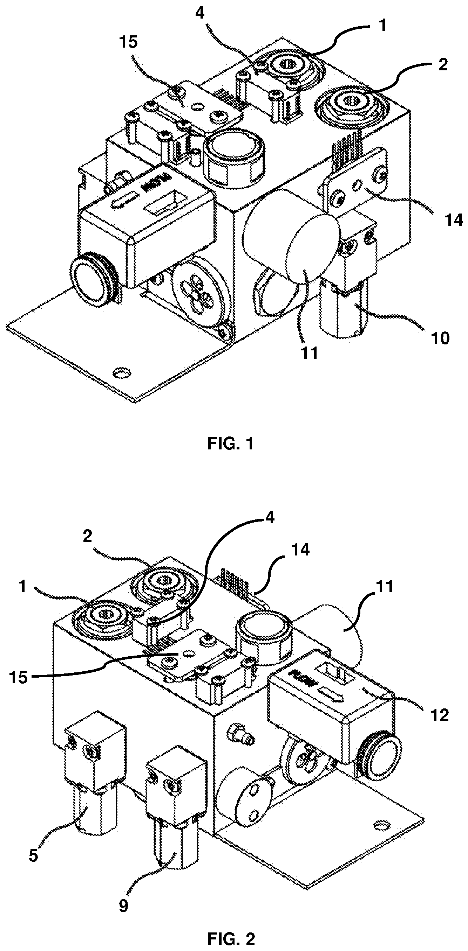

The present invention will be further described based on an example of preferred embodiment of the transport ventilator according to the present invention illustrated in figures 1 to 4.

Figure 1 shows a first perspective view of the transport ventilator of the present invention.

The ventilator comprises a monobloc body structure where the ventilator components are inserted or attached and within which the gas passageways are formed.

Figure 1 shows the outside part of the monobloc where an oxygen pressure regulating valve 1 is fixed, through which pressurized medical oxygen enters, and an air pressure regulating valve 2, through which the compressed air enters.

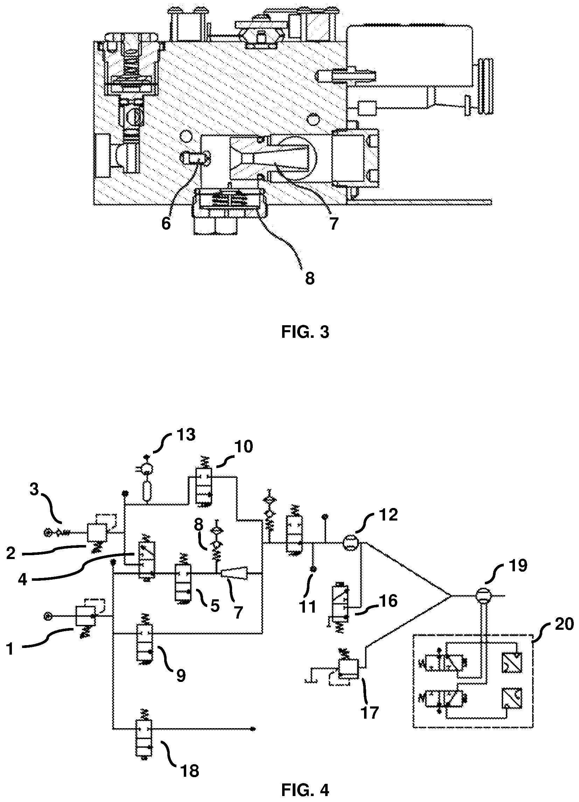

The air pressure regulating valve 2 may be associated with a one-way valve 3, which is schematically illustrated in figure 4. The one-way valve 3 prevents the return of the gas in the absence of the air source.

The compressed air can be any suitable source, such as a hospital compressed air supply network, a compressed air cylinder with a regulating valve or an air compressor.

The oxygen source can be any suitable source, such as a hospital oxygen supply network or an oxygen cylinder with a regulating valve.

As illustrated in figures 1 and 2, the ventilator can also comprise, on the outer face of the monobloc body, an air pressure measuring point 14 and an oxygen pressure measuring point 15.

A venturi system, which comprises an injector nozzle 6 and a venturi tube, uses the pressure of the entering gas to generate a vacuum which allows the dragging of ambient air. As shown in figure 3, ambient air enters through an ambient air inlet assembly formed by a one-way valve 8 associated with a filter. As shown in the cross-sectional view of figure 3, the injector nozzle 6 and the venturi system 7 are formed inside the monobloc body.

A solenoid valve 4 is provided to act as a gas selector valve for the mixture to be delivered to the patient. Thus, the solenoid valve 4 allows alternative passage of air or oxygen. As it will be explained later, the selector solenoid valve 4 allows automatic adjustment of the oxygen concentration in FiO₂.

In the preferred embodiment of the invention, the ventilator also comprises at least one proportional valve 5, preferably fixed to the face of the monobloc body. The proportional valve 5, shown in figures 2 and 4, is a pneumatic valve commanded by an electrical signal whose flow is proportional to the fed electric current.

Thus, the gas (either oxygen or air), exits the respective regulating valve 1 or 2 with the set pressure, passes through the solenoid valve 4 and continues to the proportional valve 5.

The proportional valve 5 provides the gas to the injector nozzle 6 of the venturi system 7 (see figures 3 and 4). Thereby, the gas, upon passing through the injector nozzle 6, becomes a gas jet dragging ambient air which enters the monobloc through the ambient air inlet assembly. The gas mixture that exits the venturi system 7 goes to the patient.

The transport ventilator of the present invention allows the oxygen concentration in the inspired gas to be adjusted to values between 21% and 100% by the electronic control of the selector solenoid valve 4.

When the selector solenoid valve 4 is open for only the oxygen inlet (i.e., when the solenoid valve 4 is "off"), the oxygen is directed to the injector nozzle 6 dragging the ambient air through the ambient air inlet assembly. In this situation, the gas mixture formed in the venturi tube has a minimum FiO₂ of about 35% O₂ in a higher yield flow rate (i.e., the minimum oxygen concentration achieved in the gas mixture in the venturi when the inlet gas is oxygen is at least about 35%, since the O₂ dragged is mixed with ambient air which has about 21% of oxygen). This minimum value tends to increase when there is a higher pressure at the exit of the venturi tube 7 or when a very low flow rate is set as that suitable for neonates (i.e.; when less ambient air is absorbed).

Thus, in order to achieve an O₂ concentration of less than 35% in FiO₂, the transport ventilator of the present invention allows the solenoid valve 4 to be selectively "activated" to allow the use of the compressed gas as a motive gas (i.e., the selector valve 4 starts to allow the passage of compressed air). In this situation, the O₂ that enters into the mixture is that one from ambient air which was dragged by the ambient air inlet assembly. Thus, in this situation, the FiO₂ will be 21% of O₂ from very low flow to the maximum air flow that can be inhaled.

This control of the selector solenoid valve is automatically performed by a software-based control system based on the oxygen concentration measured by an fraction of inspired oxygen sensor 11. Thus, the solenoid valve 4 allows to choose, selectively, between the passage of medical O₂ and the passage of compressed air until the measured oxygen concentration corresponds to the concentration of O₂ in the FiO₂ desired for the situation of use.

In a preferred embodiment of the ventilator of the present invention, the transport ventilator further includes a proportional oxygen valve 9 that allows the passage of oxygen directly, without passing through the injector nozzle. Preferably, the proportional valve 9 is a high flow valve.

Similarly, in the preferred embodiment, the transport ventilator includes a proportional air valve 10 that allows the passage of air directly, without passing through the injector nozzle. Preferably, the proportional valve 10 is a high flow valve.

The proportional oxygen 9 and air 10 valves have the objective of completing the current volume required for the FiO₂ adjusted in the ventilator. This control is done by a control software, which, based on the information received from the flow sensor 12, adjusts the current volume based on the measurement of the flow exiting the monobloc body.

Thus, the control software adjusts the current volume by opening and closing the proportional oxygen valves 9 and air 10.

It should be noted that the algorithm of the control software prioritizes the use of the venturi system, so that the proportional oxygen 9 and air 10 are only opened to complete the required current volume for FiO2.

Thus, in a situation where the selector solenoid valve 4 only allows the passage of air as motive gas and where, at the maximum flow provided by the venturi, the ventilator cannot ventilate with the required volume, the proportional air valve 10 and/or the proportional O₂ valve 9 open to complete the adjusted volume in the FiO₂ required.

In the preferred embodiment of the present invention, the transport ventilator is also adapted to be coupled thereto by a mini compressor or small air cylinder 13 as a source of the compressed air used as motive gas, as shown in the diagram of figure 4. The possibility of coupling the small air source, associated with the selector valve 4 and the venturi system, allows the transport ventilator to provide good autonomy, since air consumption by the venturi system is low.

It should be noted that the compressor or small cylinder could also be inserted into the monobloc body itself, increasing even further the compaction of the assembly.

In the illustrative example of figure 4, the pneumatic scheme of the transport ventilator further comprises:
- a safety valve 16, which opens in case of overpressure and reliefs the pressure of the flow delivered to the patient, thereby avoiding barotrauma;
- an expiratory valve 17, which closes, following an electric command, to direct the gas to the lung of a patient during the inspiration/ inhalation phase and opens to exhale the gas from the lung during the expiration/ exhalation phase;
- a solenoid valve 18 which may be used to allow the gas flow to be used by an accessory device such as a nebulizer or may be connected to a tube to deliver a gas jet directly to the tip of an intubation cannula (knows as - TGI / tracheal gas injection) to aid in expelling the CO2 that sometimes accumulates in the lung;
- a proximal flow sensor 19 which, when activated, monitors the gases than enter and leave the mouth of the patient; and
- an offset arrangement 20, comprising two three-way solenoid valves, intended to zero the offset of the pressure differential transducers that measure the inspiratory and expiratory flows.

Therefore, having described examples of embodiments of the present invention, it should be understood that the scope of the present invention covers other possible variations of the inventive concept described, being limited only by the content of the claims, including possible equivalents.

## Claims

1. Transport ventilator **characterized by** comprising:
a monobloc body with at least one medical oxygen inlet (1); at least one compressed air inlet (2), and at least one ambient air inlet (8);
at least one selector valve (4) integrated to the monobloc body and adapted to allow, selectively, the passage of medical oxygen or compressed air;
a venturi system, internal to the monobloc body, that includes an injector nozzle (6) and a venturi tube (7), the injector nozzle receiving the medical oxygen or the compressed air passing through the selector valve (4) and generating a jet towards the venturi tube (7), the jet dragging ambient air through the ambient air inlet (8) to generate a mixture of gases to be ventilated to a patient;
a fraction of inspired oxygen sensor (11) that measures the concentration of oxygen in the gas mixture; and
an electronic control system that controls the selector valve (4) to allow the passage of medical oxygen or compressed air based on the oxygen concentration measured by the fraction of inspired oxygen sensor (11).

2. Transport ventilator, according to claim 1, **characterized by** the fact that the selector valve is a solenoid valve (4).

3. Transport ventilator, according to claim 2, **characterized by** the fact that the transport ventilator further comprises a small compressed air source, wherein the compressed air inlet is fed by the small compressed air source coupled to the monobloc body.

4. Transport ventilator, according to claim 3, **characterized by** the fact that the small compressed air source is selected from a mini compressor coupled to the monobloc body or a small cylinder coupled to the monobloc body.

5. Transport ventilator, according to claim 1, **characterized by** the fact the medical oxygen inlet (1) is an oxygen pressure regulating valve (1), the compressed air inlet is an air pressure regulating valve (2), and the ambient air inlet is a one-way valve (8) which comprises a filter.

6. Transport ventilator, according to claim 5, **characterized by** the fact it further comprises a proportional valve (5) that receives the oxygen or compressed air which passes through the selector valve (4) e forwards it to the injector nozzle (6).

7. Transport ventilator, according to claim 6, **characterized by** the fact it further comprises:
an oxygen proportional valve (9) that allows the passage of medical oxygen from the oxygen pressure regulating valve (1) directly into the gas mixture to be ventilated;
an air proportional valve (10) that allows the passage of compressed air from the air pressure regulating valve (2) directly into the gas mixture to be ventilated;
in which the electronic control system opens and closes the oxygen proportional valve (9) and air proportional valve (10) based on the measurement of the exit flow of the gas mixture measured by a flow sensor (12).

## Patentansprüche

1. Transportventilator, **dadurch gekennzeichnet, dass** er umfasst:
einen Monoblockkörper mit wenigstens einem medizinischen Sauerstoffeinlass (1); wenigstens einem Drucklufteinlass (2) und wenigstens einem Umgebungslufteinlass (8);
wenigstens ein Auswahlventil (4), welches in den Monoblockkörper integriert ist und dazu eingerichtet ist, selektiv den Durchgang von medizinischem Sauerstoff oder Druckluft zu ermöglichen;
ein Venturi-System innerhalb des Monoblockkörpers, welches eine Einspritzdüse (6) und ein Venturi-Rohr (7) umfasst, wobei die Einspritzdüse den medizinischen Sauerstoff oder die Druckluft aufnimmt, welcher/welche durch das Auswahlventil (4) strömt, und einen Strahl in Richtung des Venturi-Rohrs (7) erzeugt, wobei der Strahl Umgebungsluft durch den Umgebungslufteinlass (8) mitzieht, um ein einem Patienten zu ventilierendes Gasgemisch zu erzeugen;
einen Sensor (11) für einen Anteil eingeatmeten Sauerstoffs, welcher die Sauerstoffkonzentration in dem Gasgemisch misst; und
ein elektronisches Steuersystem, welches das Auswahlventil (4) steuert, um den Durchgang von medizinischem Sauerstoff oder Druckluft auf Grundlage der durch den Sensor (11) für einen Anteil eingeatmeten Sauerstoffs gemessenen Sauerstoffkonzentration zu ermöglichen.

2. Transportventilator nach Anspruch 1, **gekennzeichnet durch** die Tatsache, dass das Auswahlventil ein Magnetventil (4) ist.

3. Transportventilator nach Anspruch 2, **gekennzeichnet durch** die Tatsache, dass der Transportventilator ferner eine kleine Druckluftquelle umfasst, wobei der Drucklufteinlass durch die kleine Druckluftquelle gespeist wird, welche mit dem Monoblockkörper gekoppelt ist.

4. Transportventilator nach Anspruch 3, **gekennzeichnet durch** die Tatsache, dass die kleine Druckluftquelle aus einem mit dem Monoblockkörper gekoppelten Minikompressor oder einem mit dem Monoblockkörper gekoppelten kleinen Zylinder ausgewählt ist.

5. Transportventilator nach Anspruch 1, **gekennzeichnet durch** die Tatsache, dass der medizinische Sauerstoffeinlass (1) ein Sauerstoffdruckregelventil (1) ist, der Drucklufteinlass ein Luftdruckregelventil (2) ist und der Umgebungslufteinlass ein Einwegventil (8) ist, welches einen Filter umfasst.

6. Transportventilator nach Anspruch 5, **gekennzeichnet durch** die Tatsache, dass er ferner ein Proportionalventil (5) umfasst, welches den Sauerstoff oder die Druckluft aufnimmt, welcher/welche durch das Auswahlventil (4) strömt, und ihn/sie zu der Einspritzdüse (6) weiterleitet.

7. Transportventilator nach Anspruch 2, **gekennzeichnet durch** die Tatsache, dass er ferner umfasst:
ein Sauerstoffproportionalventil (9), welches den Durchgang von medizinischem Sauerstoff aus dem Sauerstoffdruckregelventil (1) direkt in das zu ventilierende Gasgemisch ermöglicht;
ein Luftproportionalventil (10), welches den Durchgang von Druckluft aus dem Luftdruckregelventil (2) direkt in das zu ventilierende Gasgemisch ermöglicht;
wobei das elektronische Steuersystem das Sauerstoffproportionalventil (9) und das Luftproportionalventil (10) auf Grundlage der Messung des durch einen Strömungssensor (12) gemessenen Ausgangsstroms des Gasgemisches öffnet und schließt.

## Revendications

1. Ventilateur de transport **caractérisé en ce qu'**il comprend :
un corps monobloc avec au moins une entrée d'oxygène médical (1) ; au moins une entrée d'air comprimé (2), et au moins une entrée d'air ambiant (8) ;
au moins une vanne de sélection (4) intégrée au corps monobloc et adaptée pour permettre, de manière sélective, le passage d'oxygène médical ou d'air comprimé ;
un système venturi, interne au corps monobloc, qui comporte une buse d'injecteur (6) et un tube venturi (7), la buse d'injecteur recevant l'oxygène médical ou l'air comprimé passant par la vanne de sélection (4) et générant un jet vers le tube venturi (7), le jet acheminant l'air ambiant à travers l'entrée d'air ambiant (8) pour générer un mélange de gaz à ventiler vers un patient ;
une fraction de capteur d'oxygène inspiré (11) qui mesure la concentration d'oxygène dans le mélange gazeux ; et
un système de commande électronique qui commande la vanne de sélection (4) pour permettre le passage d'oxygène médical ou d'air comprimé sur la base de la concentration en oxygène mesurée par la fraction de capteur d'oxygène inspiré (11).

2. Ventilateur de transport, selon la revendication 1, **caractérisé en ce que** la vanne de sélection est une électrovanne (4).

3. Ventilateur de transport, selon la revendication 2, **caractérisé en ce que** le ventilateur de transport comprend en outre une petite source d'air comprimé, dans lequel l'entrée d'air comprimé est alimentée par la petite source d'air comprimé couplée au corps monobloc.

4. Ventilateur de transport, selon la revendication 3, **caractérisé en ce que** la petite source d'air comprimé est choisie parmi un mini compresseur couplé au corps monobloc ou un petit cylindre couplé au corps monobloc.

5. Ventilateur de transport, selon la revendication 1, **caractérisé en ce que** l'entrée d'oxygène médical (1) est une vanne de régulation de pression d'oxygène (1), l'entrée d'air comprimé est une vanne de régulation de pression d'air (2), et l'entrée d'air ambiant est une vanne unidirectionnelle (8) qui comprend un filtre.

6. Ventilateur de transport, selon la revendication 5, **caractérisé en ce qu'**il comprend en outre une vanne proportionnelle (5) qui reçoit l'oxygène ou l'air comprimé qui passe à travers la vanne de sélection (4) et l'achemine vers la buse d'injecteur (6).

7. Ventilateur de transport, selon la revendication 6, **caractérisé en ce qu'**il comprend en outre :
une vanne proportionnelle d'oxygène (9) qui permet de ventiler le passage d'oxygène médical depuis la vanne de régulation de pression d'oxygène (1) directement dans le mélange gazeux ;
une vanne proportionnelle d'air (10) qui permet de ventiler le passage d'air comprimé depuis la vanne de régulation de pression d'air (2) directement dans le mélange de gaz ;
dans lequel le système de commande électronique ouvre et ferme la vanne proportionnelle d'oxygène (9) et la vanne proportionnelle d'air (10) sur la base de la mesure du débit de sortie du mélange de gaz mesuré par un capteur de débit (12).
